# EUROPÄISCHE PATENTSCHRIFT

(11) **EP 1 235 780 B1**
(45) Veröffentlichungstag und Bekanntmachung des Hinweises auf die Patenterteilung: **18.02.2004**
(21) Anmeldenummer: 00987303.5
(22) Anmeldetag: 27.11.2000
(51) Int. Cl.: C07C 51/00

(54) **VERFAHREN ZUR HERSTELLUNG VON O-CHLORMETHYLBENZOESÄURECHLORIDEN**
METHOD FOR PRODUCING O-CHLOROMETHYL BENZOIC ACID CHLORIDES
PROCEDE DE PREPARATION DE CHLORURES D'ACIDE O-CHLOROMETHYLBENZOIQUE

(30) Priorität: 07.12.1999 DE 19958758; 19.02.2000 DE 10007695
(43) Veröffentlichungstag der Anmeldung: 04.09.2002
(73) Patentinhaber: BASF AKTIENGESELLSCHAFT, 67056 Ludwigshafen (DE)
(72) Erfinder: GÖTZ, Roland, 68809 Neulussheim (DE); GÖTZ, Norbert, 67547 Worms (DE); KEIL, Michael, 67251 Freinsheim (DE); WOLF, Bernd, 67136 Fussgönheim (DE); STEINMETZ, Adrian, 68199 Mannheim (DE); STAMM, Armin, 55268 Nieder-Olm (DE); HENKELMANN, Jochem, 68165 Mannheim (DE)
(74) Vertreter: Fitzner, Uwe, Dr.
(86) Internationale Anmeldenummer: PCT/EP2000/011810
(87) Internationale Veröffentlichungsnummer: WO 2001/042182

(56) Entgegenhaltungen:
- EP-A- 0 676 389
- WO-A-99/16743

## Beschreibung

Die vorliegende Erfindung betrifft ein Verfahren zur Herstellung von o-Chlormethylbenzoesäurechloriden der Formel I, 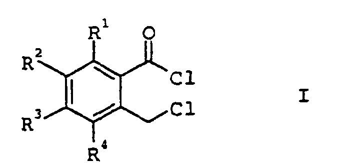 in der R¹ bis R⁴ gleich oder verschieden sein können und für Wasserstoff, C₁-C₄-Alkyl, Halogen oder Trifluormethyl stehen, durch Umsetzung von benzokondensierten Lactonen der Formel II, 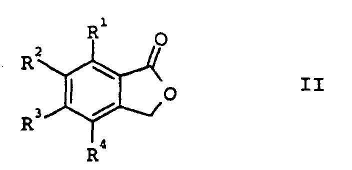 in der R¹ bis R⁴ die vorgenannte Bedeutung besitzen, mit Thionylchlorid.

o-Chlormethyl-substituierte Benzoylchloride sind wichtige Zwischenprodukte zur Herstellung von beispielsweise pestiziden Wirkstoffen wie sie in den Patentschriften EP-A 460 575, EP-A 463 488, WO-A 95/18789, WO-A 95/21154 und WO-A 97/15552 beschrieben sind.

o-Chlormethyl-substituierte Benzoylchloride können zum Beispiel durch Umsetzung von benzokondensierten Lactonen mit Thionylchlorid oder Phosgen hergestellt werden. Bei der Verwendung von Thionylchlorid vereinfacht sich die Apparatetechnik und der sicherheitstechnische Aufwand verringert sich.

In EP-A 676 389 wird die Herstellung von o-Chlormethylbenzoesäurechloriden aus benzokondensierten Lactonen mit Thionylchlorid in Gegenwart einer Stickstoff-Verbindung beschrieben. Um einen befriedigenden Umsatz zu erzielen, sind Reaktionstemperaturen von 160 bis 170°C erforderlich, bei denen sich Thionylchlorid bereits teilweise zersetzt und infolgedesssen störende Nebenprodukte gebildet werden. Weiterhin ist die Zugabe von gasförmiger Salzsäure erforderlich. Schließlich liegen die Ausbeuten zum Teil deutlich unter 90%.

In der WO-A 99/16743 wird die Umsetzung mit Thionylchlorid in Gegenwart von BF₃-Etherat und einem quartären Ammoniumsalz bei 90 bis 100°C durchgeführt. Hier erweist sich als nachteilig, daß das BF₃-Etherat sehr feuchtigkeitsempfindlich ist und weiterhin im Verlauf der Reaktion Diethylether freisetzen kann. Diethylether ist niedrigsiedend, leicht brennbar und neigt zur Bildung von Peroxiden. Durch Hydrolyse von Bortrifluorid kann weiterhin Fluorwasserstoff (Flußsäure) entstehen, der stark ätzend ist und insbesondere materialtechnische Probleme aufwirft. Für die Durchführung im technischen Maßstab sind daher spezielle Apparaturen erforderlich.

Aufgabe der vorliegenden Erfindung war es daher ein wirtschaftliches und prozeßfähiges Verfahren zur Herstellung von o-Chlormethylbenzoesäurechloriden zu finden, das die oben aufgeführten Nachteile nicht aufweist und dennoch hohe Ausbeuten liefert.

Demgemäß wurde das eingangs erwähnte Verfahren gefunden, das dadurch gekennzeichnet ist, daß man die Umsetzung in Gegenwart katalytischer Mengen eines borhaltigen Katalysators ausgewählt aus der Gruppe: Borsäure, Borsäureanhydrid, Borat, Boronsäure, Boronsäureester und katalytischer Mengen eines Ammoniumsalzes durchführt.

Zum Einsatz gelangen benzokondensierte Lactone (Phthalide) der allgemeinen Formel II 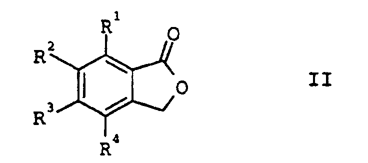 in der R¹ bis R⁴ gleich oder verschieden sein können und für Wasserstoff (H), C₁-C₄-Alkyl, Halogen (Fluor, Chlor, Brom oder Iod) oder Trifluormethyl stehen. Bevorzugt wird unsubstituiertes Phthalid eingesetzt

Als quartäre Ammoniumsalze kommen insbesondere quartäre Ammoniumhalogenide wie beispielsweise Tetraalkylammoniumchloride oder Benzyltrialkylammoniumchloride in Frage. Insbesondere geeignet sind: Tetramethylammoniumchlorid, Tetraethylammoniumchlorid, Tetrabutylammoniumchlorid, Benzyltrimethylammoniumchlorid, Benzyltriethylammoniumchlorid, Benzyltributylammoniumchlorid, N,N-Dimethylpiperidininumchlorid. Besonders bevorzugt ist Benzyltriethylammoniumchlorid.

Die quartären Ammoniumsalze werden im allgemeinen in Mengen von 0,1 bis 20 mol-% bezogen auf die eingesetzte Menge an benzokondensiertem Lacton, bevorzugt in Mengen von 0,5 bis 10 mol-%, zugegeben.

Die Reaktion wird weiterhin in Gegenwart katalytischer Mengen von Borsäure, Borsäureanhydrid, Borat, Boronsäure oder Boronsäureester durchgeführt. Als Boronsäure/ester kommen die folgenden Verbindungen in Frage: Arylboronsäure (insbesondere Phenylboronsäure), Arylboronsäure-C₁-C₄-alkylester, C₁-C₆-Alkylboronsäure oder C₁-C₆-Alkylboronsäure-C₁-C₄-alkylester. Als Borate sind insbesondere Alkaliborate wie Borax in Betracht zu ziehen.

Besonders bevorzugt wird Borsäure eingesetzt. Derartige Verfahren liefern hervorragende Ausbeuten und haben den Vorteil, daß die Reaktionsgemische frei von Fluoridionen sind. Damit kann im Verlauf der Reaktion keine Flußsäure (Fluorwasserstoff) enstehen. Im Vergleich zur analogen Reaktion mit BF₃-Derivaten als Lewis-Säure vereinfacht sich die gesamte Apparatetechnik.

Die Borsäure wird in Mengen von 0,1 bis 20 mol-% bezogen auf die eingesetzte Menge an benzokondensiertem Lacton (Phthalid), bevorzugt in Mengen von 0,5 bis 10 mol-%, zugegeben.

In Bezug auf das Phthalid II werden im allgemeinen 1 bis 1,5 Äquivalente Thionylchlorid eingesetzt.

Das Thionylchlorid kann mit den anderen Reaktionspartnern vorgelegt werden (batch-Fahrweise) oder im Verlauf der Reaktion innerhalb von vorzugsweise 1 bis 8 Stunden zudosiert werden (semibatch-Fahrweise). Weiterhin ist es möglich die Reaktion kontinuierlich durchzuführen.

Optional kann zur Beschleunigung der Ringöffnung gasförmiger Chlorwasserstoff eingeleitet werden. Bevorzugt wird jedoch auf die Einleitung von Chlorwasserstoff während der Synthese verzichtet.

Üblicherweise werden Reaktionstemperaturen von 100 bis 180°C und vorzugsweise 110 bis 150°C angewendet.

Das Verfahren wird vorzugsweise ohne Lösungsmittel durchgeführt. Es ist jedoch möglich ein gegen Thionylchlorid inertes Lösungsmittel zuzusetzen. Inerte Lösungsmittel sind z. B. aromatische Kohlenwasserstoffe wie Toluol, o-, m- oder p-Xylol oder deren Gemische, chlorierte aromatische Kohlenwasserstoffe wie Chlorbenzol oder Dichlorbenzolen oder cyclische Carbonate wie Ethylen- oder Propylencarbonat. Weiterhin ist es möglich, Thionylchlorid selbst als Lösungsmittel einzusetzen, das am Ende der Reaktion destillativ abgetrennt und erneut in den Prozeß eingeschleust werden kann.

Die Reaktion wird im allgemeinen bei Normaldruck oder bei 1 bis 10 bar Druck durchgeführt.

Die nachfolgenden Beispiele sollen das Verfahren weiter erläutern.

### Verfahrensbeispiele

### Allgemeine Vorschrift zur Herstellung von o-Chlormethylbenzoylchlorid

In einer Rührapparatur bestehend aus einem 1,6 l Doppelmantelreaktor mit aufgesetzter Intensivkühlungskaskade wurden jeweils X mol Phthalid zusammen mit dem jeweiligen Katalysatorsystem vorgelegt. 1,3 Äqivalente Thionylchlorid bezogen aufs Phthalid wurden entweder mit den anderen Komponenten vorgelegt oder während des Zeitraums von 1 bis 8 Stunden zugetropft. Danach ließ man 1 bis 15 Stunden bei Reaktionstemperatur nachrühren. Der Wertgehalt der Rohmischung wurde per GC ermittelt. In ausgewählten Beispielen wurde das Produkt bei 0,5 mbar und 75 bis 85°C durch fraktionierte Destillation isoliert.

### Beispiel 1

268 g (2 mol) Phthalid, 12,4 g (0,2 mol, 10 mol%) Borsäure und 45,4 g (0,2 mol, 10 mol%) Benzyltriethylammoniumchlorid wurden im Rührbehälter vorgelegt und auf 130°C erwärmt. Zu dieser Schmelze wurden innerhalb von 5 Stunden 310 g (2,6 mol) Thionylchlorid zugetropft. Anschließend wurde noch 5 Stunden bei 130°C nachgerührt. Der Reaktionsaustrag enthielt 97 GC-Flächen-% o-Chlormethylbenzoylchlorid.

### Beispiel 2

670 g (5 mol) Phthalid, 9,3 g (0,15 mol, 3 mol%) Borsäure und 34,1 g (0,15 mol, 3 mol%) Benzyltriethylammoniumchlorid wurden im Rührbehälter vorgelegt und auf 130°C erwärmt. Zu dieser Schmelze wurden innerhalb von 5 Stunden 774 g (6,5 mol) Thionylchlorid zugetropft. Anschließend wurde noch 5 Stunden bei 130°C nachgerührt.

Nach Destillation des Reaktionsaustrags wurden 940 g (99,4 % Ausbeute) o-Chlormethylbenzoylchlorid in einer Reinheit von 98,1 % (GC) erhalten.

## Patentansprüche

1. Verfahren zur Herstellung von o-Chlormethylbenzoesäurechloriden der Formel I, 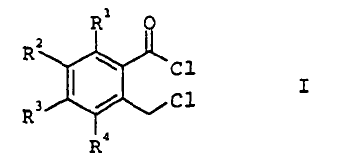 in der R¹ bis R⁴ gleich oder verschieden sein können und für Wasserstoff, C₁-C₄-Alkyl, Halogen oder Trifluormethyl stehen, durch Umsetzung von benzokondensierten Lactonen der Formel II, 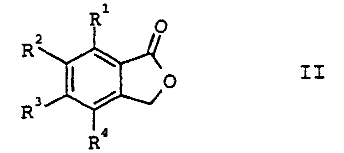 in der R¹ bis R⁴ die vorgenannte Bedeutung besitzen, mit Thionylchlorid,
**dadurch gekennzeichnet, daß** man die Umsetzung in Gegenwart
i) katalytischer Mengen eines borhaltigen Katalysators ausgewählt aus der Gruppe: Borsäure, Borsäureanhydrid, Borat, Boronsäure, Boronsäureester; und
ii) katalytischer Mengen eines quartären Ammoniumsalzes;
durchführt.

2. Verfahren gemäß Anspruch 1, **dadurch gekennzeichnet, daß** als borhaltiger Katalysator: Borsäure eingesetzt wird.

3. Verfahren gemäß Anspruch 1, **dadurch gekennzeichnet, daß** als borhaltiger Katalysator: Arylboronsäure, Arylboronsäure-C₁-C₄alkylester, C₁-C₆-Alkylboronsäure oder C₁-C₆-Alkylboronsäure-C₁-C₄-alkylester eingesetzt wird.

4. Verfahren gemäß Anspruch 1 bis 3, **dadurch gekennzeichnet, daß** der borhaltige Katalysator in einer Konzentration von 0,1 bis 20 mol-% bezogen auf das Lacton II eingesetzt wird.

5. Verfahren gemäß einem der Ansprüche 1 bis 4, **dadurch gekennzeichnet, daß** 0,1 bis 20 mol-% des quartären Ammoniumsalzes bezogen auf das Lacton II eingesetzt werden.

## Claims

1. A process for preparing o-chloromethylbenzoyl chlorides of the formula I, 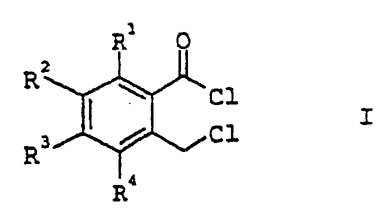 in which R¹ to R⁴ can be identical or different and are hydrogen, C₁-C₄-alkyl, halogen or trifluoromethyl,
by reacting benzo-fused lactones of the formula II, 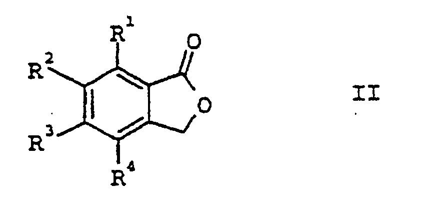 in which R¹ to R⁴ are as defined above with thionyl chloride,
which comprises carrying out the reaction in the presence of
i) catalytic amounts of a boron-containing catalyst selected from the group consisting of: boric acid, boric anhydride, borate, boronic acid, boronic esters; and
ii) catalytic amounts of a quaternary ammonium salt.

2. A process as claimed in claim 1, wherein the boron-containing catalyst used is boric acid.

3. A process as claimed in claim 1, wherein the boron-containing catalyst used is an arylboronic acid, an arylboronic acid C₁-C₄-alkyl ester, a C₁-C₆-alkylboronic acid or a C₁-C₆-alkylboronic acid C₁-C₄-alkyl ester.

4. A process as claimed in any of claims 1 to 3, wherein the boron-containing catalyst is employed in a concentration of from 0.1 to 20 mol%, based on the lactone II.

5. A process as claimed in any of claims 1 to 4, wherein the quaternary ammonium salt is employed in a concentration of from 0.1 to 20 mol%, based on the lactone II.

## Revendications

1. Procédé pour la préparation de chlorures d'o-chlorométhylbenzoyle de formule I 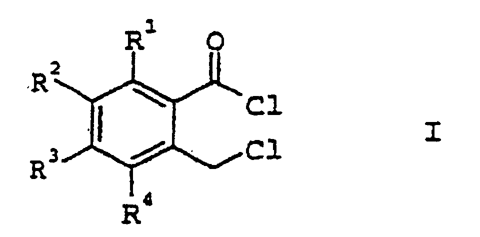 dans laquelle R¹ à R⁴ peuvent être identiques ou différents et représentent un atome d'hydrogène ou d'halogène ou un groupe alkyle en C₁-C₄ ou trifluorométhyle,
par réaction de lactones condensées avec un noyau benzénique, de formule II 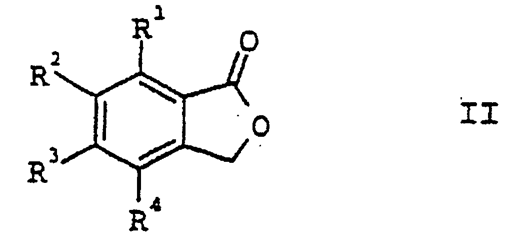 dans laquelle R¹ à R⁴ ont les significations précitées, avec du chlorure de thionyle,
**caractérisé en ce qu'**on effectue la réaction en présence
i) de quantités catalytiques d'un catalyseur contenant du bore, choisi dans le groupe constitué par : l'acide borique, l'anhydride borique, un borate, l'acide boronique, un ester d'acide boronique ; et
ii) de quantités catalytiques d'un sel d'ammonium quaternaire.

2. Procédé selon la revendication 1, **caractérisé en ce qu'**on utilise en tant que catalyseur contenant du bore : l'acide borique.

3. Procédé selon la revendication 1, **caractérisé en ce qu'**on utilise en tant que catalyseur contenant du bore : un acide arylboronique, un arylboronate d'alkyle en C₁-C₄, un acide alkyl(C₁-C₆)boronique ou un alkyl(C₁-C₆)boronate d'alkyle en C₁-C₄.

4. Procédé selon l'une quelconque des revendications 1 à 3, **caractérisé en ce que** le catalyseur contenant du bore est utilisé à une concentration de 0,1 à 20 % en moles par rapport à la lactone II.

5. Procédé selon l'une quelconque des revendications 1 à 4, **caractérisé en ce qu'**on utilise de 0,1 à 20 % en moles du sel d'ammonium quaternaire par rapport à la lactone II.
